# EUROPEAN PATENT SPECIFICATION

(11) **EP 2 394 638 B1**
(45) Date of publication and mention of the grant of the patent: **08.05.2013**
(21) Application number: 11169599.5
(22) Date of filing: 10.06.2011
(51) Int. Cl.: A61K 9/00, A61K 31/4184

(54) **New pharmaceutical combinations**
Neue pharmazeutische Verbindungen
Nouvelles combinaisons pharmaceutiques

(30) Priority: 11.06.2010 TR 201004754
(43) Date of publication of application: 14.12.2011
(60) Divisional application: 13150074.6
(73) Proprietor: Sanovel Ilaç Sanayi Ve Ticaret Anonim Sirketi, 34460 Sariyer/Istanbul (TR)
(72) Inventor: Cifter, Ümit, 34398 Istanbul (TR); Türkyilmaz, Ali, 34398 Istanbul (TR); Mutlu, Onur, 34398 Istanbul (TR); Ramazanoglu, Gaye, 34398 Istanbul (TR)
(74) Representative: Sevinç, Erkan

(56) References cited:
- EP-A1- 0 628 313
- WO-A1-97/02032
- WO-A1-2009/115301
- SHAH S U ET AL: "USE OF DIURETICS IN CARDIOVASCULAR DISEASE: (2) HYPERTENSION", POSTGRADUATE MEDICAL JOURNAL, MCMILLAN PRESS, BASINGSTOKE, GB, vol. 80, no. 943, 1 January 2004 (2004-01-01), pages 271-276, XP008040902, ISSN: 0032-5473, DOI: DOI:10.1136/PGMJ.2003.010843

## Description

### Technical Field of the Invention

The present invention relates to a novel pharmaceutical composition comprising a compound of Formula I (Compound I) or pharmaceutically acceptable salts thereof and one or more diuretics as effective components, wherein said one or more diuretics are selected from thiazide derivatives.

Furthermore, the invention relates to methods for preparing the pharmaceutical composition comprising Compund I and thiazide derivatives and its use for preventing or treating hypertension in mammals, particularly in humans.

### Background of the Invention

Hypertension affects about 20% of the adult population in developed countries. In the adult population aged 60 years or older, this percentage increases to about 60% to 70% in general. Hypertension also is associated with an increased risk of other physiological complications including stroke, myocardial infarction, atrial fibrillation, heart failure, peripheral vascular disease and renal impairment. Although a number of anti-hypertensive drugs are available in various pharmacological categories, the efficacy and safety of such drugs can vary from patient to patient, in this regard new treatments are still a desired subject.

Azilsartan which has a chemical name as 2-ethoxy-1-((2'-(5-oxo-2,5-dihydro-1,2,4-oxadiazol-3yl)-biphenyl-4-yl)methyl)-1H-benzimidazole-7-carboxylic acid (hereinafter referred as "Compound I") is a novel angiotensin II receptor antagonist and its chemical structure is shown in the Formula I.

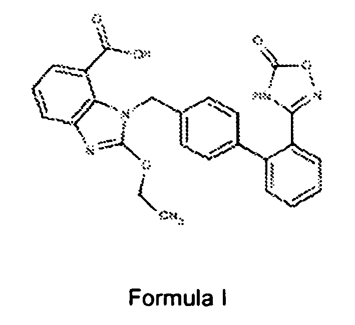

Angiotensin II receptor antagonists are used in the management of hypertension; they may have a particular role in patients who develop cough with ACE inhibitors. Some are also used in diabetic nephropathy and in the management of heart failure. They act mainly by selective blockade of AT1 receptors thus reducing the pressor effects of angiotension II. Known angiotension receptor II antagonists from the prior art are candesartan, eprosartan, irbesartan, losartan, olmesartan, telmisartan and valsartan.

Diuretics are orally administered in the treatment of oedema and hypertension. Well known diuretics are thiazides which are known moderately potent diuretics and exert their diuretic effect by reducing the reabsorbtion of electrolytes from the renal tubules, thereby increasing the excretion of sodium and chloride ions, and consequently of water.

Hydrochlorothiazide is a thiazide diuretic and its chemical name is 2H-1,2,4-Benzothiadiazine-7-sulfonamide,6-chloro-3,4-dihydro-,1,1-dioxide having the following structure (Formula II).

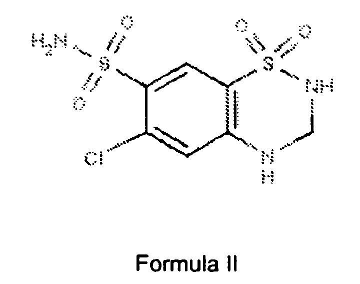

Thiazide diuretics are used in the treatment of hypertension, either alone or in combination with other antihypertensives. They are also used to treat oedema associated with heart failure and with renal and hepatic disorders. Thiazide diuretics, particularly hydrochlorothiazide may enhance the effect of other antihypertensives, particularly the first-dose hypotension that occurs with angiotensin II receptor antagonists.

It is known that co-administration of an angiotensin II receptor antagonist and a diuretic is an effective therapy for the prevention or treatment of hypertension.

However, the effects of a pharmaceutical composition comprising a specific angiotensin II receptor antagonist, such as Compound I and a diuretic selected from thiazide derivatives such as hydrochlorothiazide remain unknown.

The main challenges when combining two or more molecules in the same pharmaceutical form are (a) to guarantee the chemico-physical compatibility between the different active ingredients and/or between the active ingredients and the excipients used; and (b) to insure the therapeutical compatibility between the two active ingredients regarding their pharmacokinetic and/or pharmaceutical properties in order that the posology of the combined composition allows to obtain safe and efficient plasma levels of both pharmacological agents; and (c) to insure lower incidence of side effects.

Considering this, combinations of various drugs are investigated and found that a pharmaceutical composition containing a specific angiotensin II receptor antagonist, such as Compound I, and one or more diuretics selected from thiazide derivatives such as hydrochlorothiazide exerts excellent anti-hypertensive effects and hence useful as a preventative and/or therapeutic agent for hypertension.

Furthermore, it is known that obtaining an adequate content uniformity is an important issue especially in pharmaceutical compositions comprising more than one active ingredient. Because it is difficult to homogenize the active ingredients, particularly if they have low doses in final dosage form and some other difficulties may occur when compressing them during manufacturing process and this may cause inadequate content uniformity of the final dosage forms. As a result of this, big problems may occur during the therapy with these drugs.

It is also known in the pharmaceutical field that, when formulating a composition comprising more than one pharmaceutical active ingredient for administration to those in need of therapy, the challenge is to ensure an even distribution of the pharmaceutically active ingredients throughout the pharmaceutical excipients to ensure a proper dosage and homogeneity. Therefore, it would be desirable to provide improved processes for preparing solid oral dosage forms that have an adequate content uniformity and that disperse well upon oral administration and robust enough to remain stable during the shelf-life.

Therefore, there is need in the art for pharmaceutical compositions of Compound I or pharmaceutically acceptable salts thereof and a thiazide diuretic, particularly hydrochlorothiazide for oral administration, which has an adequate content uniformity causing a good dispersion upon oral administration and high bioavailability with improved manufacturing processes and stability and a robust final dosage form for their preparation and use thereof.

### Detailed description of the invention

The main object of the present invention is to provide new pharmaceutical compositions comprising a specific angiotensin II receptor antagonist such as Azilsartan which has a chemical name as 2-ethoxy-1-((2'-(5-oxo-2,5-dihydro-1,2,4-oxadiazol-3yl)-biphenyl-4-yl)methyl)-1H-benzimidazole-7-carboxylic acid (hereinafter reffered as "Compound I") or pharmaceutically acceptable salts thereof and one or more diuretics such as thiazide derivatives as effective components wherein the composition further comprises a disintegrant selected from the group comprising crospovidone, sodium starch glycetate, croscarmellose sodium, low substituted hydroxypropyl cellulose or mixtures thereof which overcomes the above described problems in prior art and have additive advantages over them.

According to the main object, the pharmaceutical compositions of this invention are suitable for the prevention or treating hypertension in mammals, particularly in humans.

Another object of the present invention is to obtain adequate content uniformity of dosage forms of Compound I or pharmaceutically acceptable salts thereof and one or more diuretics selected from thiazide derivatives such as hydrochlorothiazide with using adequate excipients.

Yet another object of the present invention is to provide pharmaceutical compositions of Compound I or pharmaceutically acceptable salts thereof with hydrochlorothiazide which is robust (e.g. adequate hardness, low friability) enough to be processed in high speed tablet pressing machines and shipped in low cost packages.

A further object of this invention is to provide stable pharmaceutical compositions having high bioavailability throughout the shelf-life.

Yet another object of the present invention is to provide an improved process which is simple, cost-effective and time saving for preparing the pharmaceutical composition of Compound I or pharmaceutically acceptable salts thereof and one or more thiazide diuretics such as hydrochlorothiazde.

According to the main object, said thiazide derivatives are selected from the group comprising hydrochlorothiazide, methylclothiazide, benzylhydrochlorothiazide, trichlormethiazide, cyclopenthiazide, polythiazide, ethiazide, cyclothiazide, bendroflumethiazide or hydroflumethiazide or mixtures thereof, preferably the thiazide is hydrochlorothiazide.

Investigations therefore were made to provide a fixed dose drug combination comprising an angiotensin II receptor antagonist such as Compound I or pharmaceutically acceptable salts thereof and one or more diuretics selected from thiazide derivatives such as hydrochlorothiazde. Such combination drug should display an immediate drug release profile combined with adequate stability. A fixed dose combination of drugs intended for immediate release can be prepared by either making a powder mixture or a co-granulate of the two active ingredients with the necessary excipients, normally keeping the basic formulation of the corresponding mono-drug preparation and simply adding the second drug component.

According to the present invention, the specific angiotensin II receptor antagonist such as Compound I and diuretics selected from thiazide derivatives such as hydrochlorothiazide exert better therapeutic efficacy by combined administration rather than when used separately. In addition, combinations of angiotensin II receptor antagonist and diuretics selected from thiazide deriatives such as hydrochlorothiazide are more suitable, in terms of safety or efficacy, than the administration of a single product.

However, particularly with a combination of Compound I or pharmaceutically acceptable salts thereof with hydrochlorothiazide, this approach may not feasible due to the difficulties such as Compound I is difficult to formulate and heretofore it has not been possible to make oral formulations in the form of tablets in homogenous and robust way.

We have surprisingly found that, the selection of excipients therefore has an importance to obtain an adequate content uniformity and robustness. Thus, at least one disintegrant with one or more pharmaceutically acceptable excipients can be used to obtain adequate content uniformity. The disintegrant is selected from the group comprising crospovidone, sodium starch glycolate, croscarmellose sodium, low-substituted hydroxpropyl cellulose, starch derivatives and the like or mixtures thereof; preferably the selected disintegrant is crospovidone and/or sodium starch glycolate.

Thus, crospovidone has physical and chemical properties that make it ideal for constituting the appropriate disintegrant for this invention. Because crospovidone particles have a very different appearance from those of the other disintegrants. Crospovidone particles seem to consist of aggregates of smaller particles that are fused together. This aggregation gives crospovidone a spongy, highly porous appearance and it swells very little, yet takes water into its network quite rapidly. This helps crospovidone to dissolve easily and quickly in a little amount of water and makes its disintegrating rate much faster than other related excipients.

According to this embodiment of the invention, crospovidone is present in an amount of between 0.1.0 to 30.0 % by weight, preferably in an amount of 1.0 to 20.0 % by weight of the total formulation.

In prior art, pharmaceutical compositions of angiotensin II receptor agonists with thiazide diuretics mostly have corn starch and mannitol with other common known excipients. However unmodified starch such as corn starch has poor flow characteristics and tends to increase tablet friability and capping if used in high concentrations.

We have found that, solid oral dosage forms such as tablets, when prepared with sodium starch glycolate have good storage properties. Additionally, the improved flow and lubricity characteristics of sodium starch glycolate can impart further benefit to the formulation in a very cost-effective manner. The reason for selection of sodium starch glycolate is mainly because of its high disintegrant properties besides its binding properties during granulation step in manufacturing process. So that we can use its both properties in one step.

As it is said above, the selection of excipients is very important to obtain robust final dosage forms when it is in tablet form. According to this object, investigations are made and it is found that sodium starch glycolate is one of the best excipients to obtain an adequate robustness in the final tablet dosage forms of the pharmaceutical compositions of the present invention.

Surprisingly a synergistic effect is observed over the distribution of the doses of Compound I and hydrochlorothiazide throughout other pharmaceutical excipients when crospovidone and sodium starch glycolate is used in a specific weight ratio wherein the weight ratio of crospovidone to sodium starch glycolate is between 30:1 to 1:100 by weight, preferably it is 20:1 to 1:10 by weight, more preferably it is 10:1 to 1:1 by weight of total composition. Thus, a proper dosage and homogeneity is ensured and the final dosage forms have high and adequate content uniformity. According to this embodiment, the final dosage form of the pharmaceutical composition of Compound I and hydrochlorothiazide has a content uniformity of less than 2.0% RSD (Relative Standard Deviation), preferably less than 1.0 % RSD.

According to another embodiment of the present invention, the pharmaceutical composition further comprise one or more pharmaceutical acceptable excipients which are selected form the group comprising fillers & diluents, binders, lubricants, glidants, coloring agents, coating agents or mixtures thereof.

In one embodiment, suitable fillers & diluents are selected from the group comprising microcrystalline cellulose, lactose, sucrose, glucose, sorbitol, inorganic salts, dibasic calcium phosphate dihydrate and the like or mixtures thereof; preferably the filler & diluents is microcrystalline cellulose.

Besides, it has found that, magnesium stearate has some disadvantages despite being a good lubricant and because of this it is used in small quantities during drug manufacturing process. Magnesium stearate is practically insoluble in water and because of this hydrophobic characteristic it may retard the dissolution of a drug from a solid dosage form such as tablet or capsule. Tablet and especially capsule dissolution is sensitive to both the amount of magnesium stearate in the formulation and the blending time. Blending time should be limited. Long blending times can result in the formulation of hydrophobic powder beds that do not disperse easily and overblending can cause compaction problems. Tablet dissolution rate and crushing strength decreased as the time of blending increased; and magnesium stearate may also increase tablet friability. Blending times with magnesium stearate should therefore be carefully controlled.

Thus, sodium stearyl fumarate is found to be extremely effective lubricant and less hydrophobic than magnesium stearate and has a less retardant effect on tablet dissolution than magnesium stearate. Sodium stearyl fumarate also doesn't have the over blending problems seen with magnesium stearate.

According to above embodiments the pharmaceutical composition of the present invention is free of magnesium stearate.

As it is mentioned above, one of the main object of the invention is to develop pharmaceutical compositions having optimal mechanical strength. The present invention addresses this need and discloses formulations which have a good mechanical strength. These tablets are robust (e.g., low friability, adequate hardness) enough to be processed in high speed tablet pressing machines and shipped in low cost packages, and at the same time retain good dissolution properties. These pharmaceutical compositions have an adequate bioavailability and stable throughout the shelf-life.

Surprisingly it has found that when microcrystalline cellulose and sodium stearyl fumarate is used together with Compound I or pharmaceuticaly acceptable salts thereof with hydrochlorothiazide, the pharmaceutical compositions of this invention has better storage stability and results a synergistic effect over mechanical strength such as having a better compressibility with less friability. Thus, robust tablet formulations are obtained in the final dosage forms, when the weight ratio of microcrystalline cellulose to sodium stearyl fumarate is between 100:1 and 1:100 by weight, preferably when it is between 50:1 and 1:10 by weight, more preferably it is 35:1 and 1:1 by weight of the total formulation; said amount makes it possible to significantly improve compressibility and reduce friability. Higher quantities may have negative mechanichal strength of the formulation and lower quantities may worsen the stability.

According to this embodiment the final tablet dosage forms have a hardness of between 5 to 300 Newton, preferably have a hardness of between 20 to 150 Newton and the friability of the final tablet dosage forms is less than 1%. The disintegration time of the final dosage forms are less than 2 minutes.

Furthermore, the pharmaceutical compositions of the present invention obtained are stable both to the manufacturing process and during storage, e.g a long-term shelf-life of 24 months or more at ambient temperature and in its original packaging, e.g. sealed aluminium blister packs.

In another embodiment, suitable binders are slected from the group comprising starches such as pregelatinized or modified, cellulose derivatives such as hydroxypropyl methyl cellulose, hydroxypropyl cellulose, carboxy methyl cellulose, methyl cellulose; polyvinylpyrrolidone and the like or mixtures thereof.

Acording to the embodiments described above, suitable lubricants is selected from the group comprising sodium stearyl fumarate, polyethylene glycol, stearic acid, metal stearates, talc, waxes, boric acid, hydrogenated vegetable oils, sodium chloride benzoate and acetate, sodium or magnesium lauryl sulfate and the like or mixtures thereof; preferably the lubricant is sodium stearyl fumarate.

According to one embodiment suitable glidants are selected from the group comprising colloidal silicon dioxide; silicates such as aluminium, calcium and magnesium; talc and the like or mixtures thereof; preferably the glidant is colloidal silicon dioxide.

According to one embodiment suitable coloring agents are selected from the group comprising ferric oxide (red, yellow, black or mixtures) and Food& Drug Cosmetic Dyes and the like or mixtures thereof.

According to one embodiment suitable coating agents are selected from the group comprising hydroxypropyl cellulose, hydroxypropyl methyl cellulose, methyl cellulose, polyethylene glycol, titanium dioxide, polyvinyl acetate phthalate, hydroxyproplyl methylcellulose phthalate, methacrylic acid copolymers and the like or mixtures thereof.

In one aspect, the pharmaceutical composition of Compound I is present in an amount of between 0.1 to 60.0 % by weight, preferably present in an amount of between 0.1 to 50.0 % by weight of the total composition, and hyrochlorothiazide is present in an amount of 0.1 to 40.0 % by weight of total composition.

In a preffered embodiment, the pharmaceutical composition of Compound I or a pharmaceutically acceptable salt is in the form of medoxomil salt.

In the present invention, the specific angiotensin II receptor antagonist, such as Compound I and diuretics selected from thiazides such as hydrochlorothiazide are simultaneously administered, or separately or sequentially administered as described above.

In particular, the dosage form of the present invention is a solid dosage form such as tablets, capsules, powders, sachets, etc. The preferred dosage form is in tablet or capsule form.

Yet a further object of the present invention is to provide a pharmaceutical composition in the form of a bilayer tablet comprising Compound I or pharmaceutically acceptable salts in one layer and hydrochlorothiazide in second layer.

Furthermore, the bilayer tablet dosage form may comprise a coating.

The pharmaceutical compositions of the present invention may be prepared by conventional technology well known to those skilled in the art such as wet granulation, dry granulation and direct compression and the like.

The preffered wet granulation process of the present invention for preparing the pharmaceutical composition of Compound I or pharmaceutical acceptable salts thereof and hydrocholorothiazide comprises the following steps;
a. dissolving Compound I and hydrochlorothiazide with half part of sodium starch glycolate and half part of crospovidone in organic solvent to form a solution,
b. while the solution is mixing microcrystalline cellulose is added and blended in a high-shear granulator to form granules,
c. sieving and drying the wet granules and milling the dried granules,
d. adding colloidal silicon dioxide and the rest of sodium starch glycolate and crospovidone and mixing them,
e. adding sodium stearyl fumarate to this mixture and blending them until obtaining a homogenous powder mixture,
f. compressing the blended mixture to form tablets or filling the powder
   mixture into capsules.

Another preffered wet granulation process of the present invention for preparing the pharmaceutical composition of Compound I or pharmaceutical acceptable salts thereof and hydrocholorothiazide comprises the following steps;
a. dissolving Compound I and hydrochlorothiazide with microcrystalline cellulose, half part of sodium starch glycoloate and half part of crospovidone in organic solvent to form a solution in a fluid- bed granulator,
b. sieving and drying the wet granules and milling the dried granules,
c. adding colloidal silicon dioxide and the rest of sodium starch glycolate and crospovidone and mixing them,
d. adding sodium stearyl fumarate to this mixture and blending them until obtaining a homogenous powder mixture,
e. compressing the blended mixture to form tablets or filling the powder
   mixture into capsules.

The preffered dry granulation process of the present invention for preparing the pharmaceutical composition of Compound I or pharmaceutical acceptable salts thereof and hydrocholorothiazide comprises the following steps;
a. mixing Compound I and hydrochlorothiazide with half part of microcrystalline cellulose, half part of sodium starch glycoloate, half part of crospovidone and half part of colloidal silicon dioxide,
b. pressing them with the help of a compactor,
c. sieving these powder mixture,
d. adding the rest of microcrystalline cellulose, sodium starch glycoloate, crospovidone and colloidal silicon dioxide and mixing them
e. adding sodium stearyl fumarate to this mixture and blending them until obtaining a homogenous powder mixture,
f. compressing the blended mixture to form tablets or filling the powder
   mixture into capsules.

The preferred direct compression process of the present invention for preparing the pharmaceutical composition of Compound I or pharmaceutical acceptable salts thereof and hydrocholorothiazide comprises the following steps;
a. mixing Compound I and hydrochlorothiazide with microcrystalline cellulose, sodium starch glycoloate, crospovidone and colloidal silicon dioxide for 15 to 30 min.
b. adding sodium stearyl fumarate to this mixture and blending them until obtaining a homogenous powder mixture,
c. compressing the final powder mixture to form tablets or filling the powder mixture into capsules.

The pharmaceutical compositions of this invention are suitable for preventing or treating hypertension in mammals, particularly in humans.

According to main objective of the present invention to obtain an adequate content uniformity with robust final dosage forms, this oral pharmaceutical composition has been designed, made up of the following:
a. 0.1 to 60.0 % of compound I or pharmaceutically acceptable salts thereof
b. 0.1 to 40.0 % of hydrochlorothiazide
c. 1.0 to 90.0 % of microcrystalline cellulose
d. 0.1 to 30.0 % of crospovidone
e. 0.01 to 50.0 % of sodium starch glycolate
f. 0.01 to 20.0 % of sodium stearyl fumarate
g. 0.01 to 15.0 % of colloidal silicon dioxide

This invention is further defined by reference to the following example. Although the example is not intended to limit the scope of the present invention, it should be considered in the light of the description detailed above. It will be apparent to those skilled in the art that many modifications, both to materials and methods, may be practiced without departing from the scope of the invention.

### Example 1

a. 4.0 % of compound I or pharmaceutically acceptable salts thereof
b. 5.0 % of hydrochlorothiazide
c. 64,5 % of microcrystalline cellulose
d. 20.0 % of crospovidone
e. 4.0 % of sodium starch glycolate
f. 2.0 % of sodium stearyl fumarate
g. 0,5 % of colloidal silicon dioxide

The formulation of this example is manufactured according to the process described above in the description.

### Example 2

a. 4.0 % of compound I or pharmaceutically acceptable salts thereof
b. 10.0 % of hydrochlorothiazide
c. 64.0 % of microcrystalline cellulose
d. 15 % of crospovidone
e. 4 % of sodium starch glycolate
f. 2.0 % of sodium stearyl fumarate
g. 1.0 % of colloidal silicon dioxide

The formulation of this example is manufactured according to the process described above in the description.

### Example 3

a. 25.0 % of compound I or pharmaceutically acceptable salts thereof
b. 12.50 % of hydrochlorothiazide
c. 40.50 % of microcrystalline cellulose
d. 15 % of crospovidone
e. 4 % of sodium starch glycolate
f. 2.0 % of sodium stearyl fumarate
g. 1.0 % of colloidal silicon dioxide

The formulation of this example is manufactured according to the process described above in the description.

### Example 4 (Content uniformity test)

The pharmaceutical composition of this present invention (Example 1), was tested for the content uniformity against a reference product which is including mannitol, corn starch and magnesium stearate as some of the excipients instead of sodium starch glycolate, crospovidone, microcrystalline cellulose and sodium stearyl fumarate. The results are shown below in Table 1.

**Table 1 : Content Uniformity test results (%)**

| ***Sample No*** | ***Example 1 Compound I content*** | ***Example 1 Hydrochlorothiazide content*** | ***Reference Product Compound I content*** | ***Reference Product Hydrochlorothiazide content*** |
|---|---|---|---|---|
| **1** | 101,18 | 101,53 | 108,41 | 104,54 |
| **2** | 99,63 | 99,52 | 97,58 | 89,73 |
| **3** | 100,45 | 100,08 | 95,55 | 102,11 |
| **4** | 98,52 | 101,09 | 97,32 | 96,03 |
| **5** | 100,15 | 98,95 | 103,8 | 93,5 |
| **6** | 99,66 | 98,75 | 95,19 | 96,2 |
| **7** | 99,75 | 101,25 | 87,66 | 91,8 |
| **8** | 98,89 | 99,89 | 96,84 | 97,15 |
| **9** | 98,95 | 100,05 | 106,6 | 100,5 |
| **10** | 101,3 | 101,07 | 100,2 | 96,2 |
| | | | | |
| **mean** | 99,848 | 100,218 | 98,915 | 96,776 |
| **SD** | 0,94 | 0,98 | 6,10 | 4,58 |
| **RSD %** | 0,94 | 0,98 | 6,17 | 4,74 |
| **Min.** | 98,52 | 98,75 | 87,66 | 89,73 |
| **Max.** | 101,3 | 101,53 | 108,41 | 104,54 |

**Reference Product**: Compound I, Hydrochlorothiazide as active ingredients and mannitol, corn starch, povidone, magnesium stearate and colloidal silicon dioxide as inactive ingredients.

### Example 5 (Dissolution profile test)

The pharmaceutical composition of this present invention (Example 1), was tested by its dissolution profile in phosphate buffer at pH 7.8 and 37°C using a USP apparatus 2 rotating at 50 RPM against the reference product which is mentioned in Example 4. The results are shown below in Table 2.

**Table 2 : Dissolution profile test results (%)**

| ***Time* (*min.)*** | ***Example 1 Compound I content (%)*** | ***Example 1 Hydrochlorothiazide content* (%)** | ***Reference Product Compound I content (%)*** | ***Reference Product Hydrochlorothiazide content* (%)** |
|---|---|---|---|---|
| 5 | 75 | 80 | 51 | 60 |
| 10 | 85 | 88 | 72 | 78 |
| 15 | 90 | 92 | 80 | 83 |
| 20 | 95 | 95 | 85 | 88 |
| 30 | 97 | 98 | 92 | 94 |
| 45 | 99 | 100 | 95 | 96 |
| 60 | 101 | 101 | 98 | 99 |

### Example 6 (Disintegration test)

The pharmaceutical composition of this present invention (Example 1, 2 and 3), was tested by its disintegration against the reference product which is mentioned in Example 4. The results are shown below in Table 3.

**Table 3 : Disintegration test results (min.)**

| | ***Example 1*** | ***Example 2*** | ***Example* 3** | **Reference product** |
|---|---|---|---|---|
| ***Time (min.)*** | 1.50 | 1.60 | 1.75 | 3.50 |

## Claims

1. A pharmaceutical composition comprising 2-ethoxy-1-((2'-(5-oxo-2,5-dihydro-1,2,4-oxadiazol-3yl)-biphenyl-4-yl)methyl)-1H-benzimidazole-7-carboxylic acid (Compound I) or pharmaceutically acceptable salts thereof and one or more diuretics as effective components, wherein said one or more diuretics are selected from thiazide derivatives, and the composition further comprises a disintegrant selected from the group comprising crospovidone, sodium starch glycolate, croscarmellose sodium, low-substituted hydroxpropyl cellulose or mixtures thereof.

2. The pharmaceutical composition according to claim 1, wherein the thiazide derivatives are selected from the group comprising hydrochlorothiazide, methylclothiazide, benzylhydrochlorothiazide, trichlormethiazide, cyclopenthiazide, polythiazide, ethiazide, cyclothiazide, bendroflumethiazide or hydroflumethiazide or mixtures thereof, preferably the thiazide is hydrochlorothiazide.

3. The pharmaceutical composition according to claims 1 and 2, wherein the Compound I or pharmaceutically acceptable salts thereof is present in an amount of between 0.1 and 60.0 % by weight and hydrochlorothiazide is present in an amount of 0.1 and 40.0 % by weight.

4. The pharmaceutical composition according to claims 1 to 3, wherein the pharmaceutically acceptable salt of Compound I is medoxomil.

5. The pharmaceutical composition according to claim 1, wherein the disintegrant is crospovidone and/or sodium starch glycolate.

6. The pharmaceutical composition according to claim 5, wherein crospovidone is present in an amount of between 0.10 to 30.0 % by weight of total composition.

7. The pharmaceutical composition according to claims 1 to 6, wherein the weight ratio of crospovidone to sodium starch glycolate is between 30:1 and 1:100 by weight of total composition.

8. The pharmaceutical composition according to claim 1, wherein one or more excipients are selected from the group comprising fillers & diluents, lubricants, glidants, binders, coloring agents, coating agents or mixtures thereof.

9. The pharmaceutical composition according to claim 8, wherein the filler & diluent is selected from the group comprising microcrystalline cellulose, lactose, sugars, inorganic salts, dibasic calcium phosphate dihydrate and the like or mixtures thereof; preferably the filler & diluents is microcrystalline cellulose.

10. The pharmaceutical composition according to claim 8, wherein the lubricant is selected from the group comprising sodium stearyl fumarate, polyethylene glycol, stearic acid, metal stearates, talc, boric acid, hydrogenated vegetable oils, sodium chloride benzoate and acetate, sodium or magnesium lauryl sulfate and the like or mixtures thereof; preferably the lubricant is sodium stearyl fumarate.

11. The pharmaceutical composition according to claims 9 and 10, wherein the weight ratio of microcrystalline cellulose to sodium stearyl fumarate is between 100:1 and 1:100 by weight of the total formulation.

12. The pharmaceutical composition according to claim 8, wherein the glidant is selected from the group comprising colloidal silicon dioxide; silicates such as aluminium, calcium and magnesium; talc and the like or mixtures thereof; preferably the glidant is colloidal silicon dioxide.

13. The pharmaceutical composition according to any preceding claims, comprising;
a. 0.1 to 60.0 % of compound I or pharmaceutically acceptable salts thereof
b. 0.1 to 40.0 % of hydrochlorothiazide
c. 1.0 to 90.0 % of microcrystalline cellulose
d. 0.1 to 30.0 % of crospovidone
e. 0.01 to 50.0 % of sodium starch glycolate
f. 0.01 to 20.0 % of sodium stearyl fumarate
g. 0.01 to 15.0 % of colloidal silicon dioxide.

14. The pharmaceutical composition according to claim 13, wherein the pharmaceutical composition is free of magnesium stearate.

15. The pharmaceutical composition according to any preceding claims, wherein the final dosage form is orally administrated in the form of tablets, bilayer tablets, capsules, powders or sachets.

16. The pharmaceutical composition according to claim 15, wherein the final dosage form is a tablet.

17. The pharmaceutical composition according to claim 16, wherein the tablet optionally comprise a coating layer.

18. The pharmaceutical composition according to claim 16, wherein the tablet is in the form of a bilayer tablet having the compound I or pharmaceutically acceptable salts thereof in one layer and hydrochlorothiazide in second layer.

19. The pharmaceutical composition according to claim 18, wherein the bilayer tablet optionally comprise a coating layer.

20. The pharmaceutical composition according to claim 15, wherein the final dosage form is a capsule.

21. The pharmaceutical composition according to claims 1 to 20, wherein the final dosage form has a content uniformity of less than 2.0% RSD (Relative Standard Deviation), preferably less than 1.0 % RSD.

22. The pharmaceutical composition according to claims 1 to 19, wherein the hardness of the tablet is between 5 to 300 Newton, preferably it is between 20 to 150 Newton.

23. The pharmaceutical composition according to claims 1 to 19, wherein the friability of the tablet is less than 1 %.

24. The pharmaceutical composition according to any preceding claims, wherein the pharmaceutical composition has a long term shelf-life of 24 months or more at ambient temperature, in its original packaging.

25. A wet granulation process for preparing the pharmaceutical composition according to any preceding claims, comprising the following steps;
a. dissolving Compound I and hydrochlorothiazide with half part of sodium starch glycolate and half part of crospovidone in organic solvent to form a solution,
b. while the solution is mixing microcrystalline cellulose is added and blended in a high-shear granulator to form granules,
c. sieving and drying the wet granules and milling the dried granules,
d. adding colloidal silicon dioxide and the rest of sodium starch glycolate and crospovidone and mixing them,
e. adding sodium stearyl fumarate to this mixture and blending them until obtaining a homogenous powder mixture,
f. compressing the blended mixture to form tablets or filling the powder mixture into capsules.

26. A wet granulation process for preparing the pharmaceutical composition according to any preceding claims, comprising the following steps;
a. dissolving Compound I and hydrochlorothiazide with microcrystalline cellulose, half part of sodium starch glycoloate and half part of crospovidone in organic solvent to form a solution in a fluid- bed granulator,
b. sieving and drying the wet granules and milling the dried granules,
c. adding colloidal silicon dioxide and the rest of sodium starch glycolate and crospovidone and mixing them,
d. adding sodium stearyl fumarate to this mixture and blending them until obtaining a homogenous powder mixture,
e. compressing the blended mixture to form tablets or filling the powder mixture into capsules.

27. A dry granulation process for preparing the pharmaceutical composition according to any preceding claims, comprising the following steps;
a. mixing Compound I and hydrochlorothiazide with half part of microcrystalline cellulose, half part of sodium starch glycoloate, half part of crospovidone and half part of colloidal silicon dioxide,
b. pressing them with the help of a compactor,
c. sieving these powder mixture,
d. adding the rest of microcrystalline cellulose, sodium starch glycoloate, crospovidone and colloidal silicon dioxide and mixing them
e. adding sodium stearyl fumarate to this mixture and blending them until obtaining a homogenous powder mixture,
f. compressing the blended mixture to form tablets or filling the powder mixture into capsules.

28. A direct compression process for preparing the pharmaceutical composition according to any preceding claims, comprising the following steps;
a. mixing Compound I and hydrochlorothiazide with microcrystalline cellulose, sodium starch glycoloate, crospovidone and colloidal silicon dioxide for 15 to 30 min.
b. adding sodium stearyl fumarate to this mixture and blending them until obtaining a homogenous powder mixture,
c. compressing the final powder mixture to form tablets or filling the powder mixture into capsules.

29. The pharmaceutical composition according to any preceding claims, for preventing or treating hypertension in mamals, particulary in humans.

## Patentansprüche

1. Pharmazeutische Zusammensetzung umfassend: 2-Ethoxy-1-((2'-(5-oxo-2,5-dihydro-1,2,4-oxadiazol-3xl)-biphenyl-4-yl)methyl)-1H-benzimidazol-7-carboxylsäure (Zusammensetzung I) oder pharmazeutisch akzeptable Salze davon und eines oder mehrere Diuretica als wirksame Komponenten, wobei die eine oder mehrere Diuretica ausgewählt sind aus Thiazid-Derivaten, und wobei die Zusammensetzung ferner umfasst Zersetzungsmittel ausgewählt aus der Gruppe umfassend Crospovidon, Natriumstärkeglycolat, Croscarmellose-Natrium, niedrig substituierte Hydropropylcellulose oder Mischungen davon.

2. Pharmazeutische Zusammensetzung nach Anspruch 1, worin die Thiazid-Derivate ausgewählt sind aus der Gruppe umfassend Hydrochlorothiazid, Methylclothiazid, Benzylhydrochlorothiazid, Trichlormethiazid, Cyclopenthiazid, Polythiazid, Ethiazid, Cyclothiazid, Bendroflumethiazid oder Hydroflumethiazid oder Mischungen davon, vorzugsweise ist das Thiazid Hydrochlorothiazid.

3. Pharmazeutische Zusammensetzung nach den Ansprüchen 1 und 2, worin die Zusammensetzung I oder die pharmazeutisch akzeptablen Salze davon in einer Menge zwischen 0,1 und 60,0 Gew.-% vorhanden sind, und dass das Hydrochlorothiazid in einer Menge von 0,1 und 40,0 Gew.-% vorhanden ist.

4. Pharmazeutische Zusammensetzung nach den Ansprüchen 1 bis 3, worin das pharmazeutisch akzeptable Salz der Zusammensetzung I Medoxomil ist.

5. Pharmazeutische Zusammensetzung nach Anspruch 1, worin das Zersetzungsmittel Crospovidon und/oder Natriumstärkeglycolat ist.

6. Pharmazeutische Zusammensetzung nach Anspruch 5, worin das Crospovidon in einer Menge zwischen 0,10 bis 30,0 Gew.-% der gesamten Zusammensetzung vorhanden ist.

7. Pharmazeutische Zusammensetzung nach den Ansprüchen 1 bis 6, worin das Gewichtsverhältnis des Crospovidon zu dem Natriumstärkeglycolat zwischen 30:1 und 1:100 des Gewichts der gesamten Zusammensetzung liegt.

8. Pharmazeutische Zusammensetzung nach Anspruch 1, wobei die Zusammensetzung ferner umfasst einer oder mehrere Trägerstoffe aus der Gruppe umfassend Füllstoffe und Verdünnungsstoffe, Schmierstoffe, Gleitmittel, Bindemittel, Farbstoffe, Beschichtungsmittel und Mischungen davon.

9. Pharmazeutische Zusammensetzung nach Anspruch 8, worin der Füllstoff und das Verdünnungsmittel ausgewählt sind aus der Gruppe bestehend aus mikrokristalline Cellulose, Laktose, Zucker, anorganische Salze, zweibasisches Calciumphosphatdihydrat und dergleichen oder Mischungen davon; vorzugsweise ist der Füllstoff und das Verdünnungsmittel mikrokristalline Cellulose.

10. Pharmazeutische Zusammensetzung nach Anspruch 8, worin das Gleitmittel ausgewählt ist aus der Gruppe umfassend Natriumstearylfumarat, Polyethylenglycol, Stearinsäure, Metallstearate, Talk, Borsäure, hydrierte Pflanzenöle, Natriumchloridbenzoat und Acetat, Natrium oder Magnesiumlaurylsulfat und dergleichen oder Mischungen davon; vorzugsweise ist das Gleitmittel Natriumstearylfumarat.

11. Pharmazeutische Zusammensetzung nach den Ansprüchen 9 und 10, worin das Gewichtsverhältnis der mikrokristallinen Cellulose zu dem Natriumstearylfumarat zwischen 100:1 und 1:100 Gew.-% der gesamten Formulierung beträgt.

12. Pharmazeutische Zusammensetzung nach Anspruch 8, worin das Gleitmittel ausgewählt ist aus der Gruppe bestehend aus kolloidalem Siliciumdioxid; Silicaten, beispielsweise Aluminium, Calcium und Magnesium; Talk und dergleichen oder Mischungen davon; vorzugsweise ist das Gleitmittel kolloidales Siliciumdioxid.

13. Pharmazeutische Zusammensetzung nach einem der vorhergehenden Ansprüche, umfassend:
a. 0,1 bis 60,0 % der Zusammensetzung I oder der pharmazeutisch akzeptablen Salze davon
b. 0,1 bis 40,0 % Hydrochlorothiazid
c. 1,0 bis 90,0 % mikrokirstalline Cellulose
d. 0,1 bis 30,0 % Crospovidon
e. 0,01 bis 50,0 % Natriumstärkeglycolat
f. 0,01 bis 20,0 % Natriumstearylfumarat
g. 0,01 bis 15,0 % kolloidales Siliciumdioxid.

14. Pharmazeutische Zusammensetzung nach Anspruch 13, worin die pharmazeutische Zusammensetzung frei von Magnesiumstearat ist.

15. Pharmazeutische Zusammensetzung nach einem der vorhergehenden Ansprüche, worin die endgültige Verabreichungsform in Form von Tabletten, zweischichtigen Tabletten, Kapseln, Pulvern oder Portionsbeuteln oral verabreicht wird.

16. Pharmazeutische Zusammensetzung nach Anspruch 15, worin die endgültige Verabreichungsform eine Tablette ist.

17. Pharmazeutische Zusammensetzung nach Anspruch 16, worin die Tablette wahlweise eine Überzugsschicht aufweist.

18. Pharmazeutische Zusammensetzung nach Anspruch 16, worin die Tablette in der Form einer zweilagigen Tablette ist, die die Zusammensetzung I oder die pharmazeutisch akzeptablen Salze davon in einer Schicht und Hydrochlorothiazid in der zweiten Schicht hat.

19. Pharmazeutische Zusammensetzung nach Anspruch 18, worin die zweischichtige Tablette wahlweise eine Überzugsschicht aufweist.

20. Pharmazeutische Zusammensetzung nach Anspruch 15, worin die endgültige Verabreichungsform eine Kapsel ist.

21. Pharmazeutische Zusammensetzung nach den Ansprüchen 1 bis 20, worin die endgültige Verabreichungsform eine inhaltliche Ungleichmäßigkeit von weniger als 2,0 % RSD (Relative Standard Deviation = relative Standardabweichung), vorzugsweise weniger als 1,0 % RSD hat.

22. Pharmazeutische Zusammensetzung nach den Ansprüchen 1 bis 19, worin die Härte der Tablette zwischen 5 bis 300 Newton, vorzugsweise zwischen 20 bis 150 Newton, liegt.

23. Pharmazeutische Zusammensetzung nach den Ansprüchen 1 bis 19, worin die Hochfestigkeit der Tablette weniger als 1 % beträgt.

24. Pharmazeutische Zusammensetzung nach einem der vorhergehenden Ansprüche, worin die pharmazeutische Zusammensetzung eine Langzeitlebensdauer von 24 Monaten oder mehr bei Umgebungstemperatur in ihrer Originalverpackung hat.

25. Nass-Granulierverfahren zur Zubereitung der pharmazeutischen Zusammensetzung nach einem der vorhergehenden Ansprüche umfassend die folgenden Schritte:
a. Auflösen der Zusammensetzung I und von Hydrochlorothiazid mit hälftigem Anteil von Natriumstärkeglycolat und hälftigem Anteil von Crospovidon in einem organischen Lösungsmittel, um eine Lösung zu bilden,
b. während die Lösung sich mischt, Hinzugegeben von mikrokristalliner Cellulose und Abmischen in einem Granulator mit hohem Schervermögen, um Granulate zu bilden,
c. Absieben und Trocknen der nassen Granulate und Zermahlen der getrockneten Granulate,
d. Hinzugeben von kolloidalem Siliciumdioxid und dem Rest des Natriumstärkeglycolats und des Crospovidon und Mischung derselben,
e. Hinzugeben von Natriumstearylfumarat zu dieser Mischung und Abmischen derselben bis eine homogene Pulvermischung erhalten wird,
f. Komprimieren der abgemischten Mischung, um Tabletten zu bilden oder Abfüllen der Pulvermischung in Kapseln.

26. Nass-Granulierverfahren zur Zubereitung der pharmazeutischen Zusammensetzung nach einem der vorhergehenden Ansprüche umfassend die folgenden Schritte:
a. Auflösen der Zusammensetzung I und von Hydrochlorothiazid mit mikrokristalliner Cellulose, einem hälftigen Anteil des Natriumstärkeglycolats und einem hälftigen Anteil von Crospovidon in einem organischen Lösungsmittel, um eine Lösung in einem Fluidbett-Granulator zu bilden,
b. Absieben und Trocknen der nassen Granulate und Zermahlen der getrockneten Granulate,
c. Hinzugeben von kolloidalem Siliciumdioxid und dem Rest des Natriumstärkeglycolats und des Crospovidon und Mischung derselben,
d. Hinzugeben von Natriumstearylfumarat zu dieser Mischung und Abmischen derselben bis eine homogene Pulvermischung erhalten wird,
e. Komprimieren der abgemischten Mischung, um Tabletten zu bilden oder Abfüllen der Pulvermischung in Kapseln.

27. Trocken-Granulierverfahren zur Zubereitung der pharmazeutischen Zusammensetzung nach einem der vorhergehenden Ansprüche umfassend die folgenden Schritte:
a. Mischen der Zusammensetzung I und von Hydrochlorothiazid mit einem halben Anteil mikrokristalliner Cellulose, einem halben Anteil von Natriumstärkeglycolat, einem halben Anteil von Crospovidon und einem halben Teil von kolloidalem Siliciumdioxid,
b. Verpressen derselben mit Hilfe eines Kompaktierers,
c. Absieben dieser Pulvermischung,
d. Hinzugeben des Restes der mikrokristallinen Cellulose, des Natriumstärkeglycolats, des Crospovidons und des kolloidalem Siliciumdioxids und Mischung derselben,
e. Hinzugeben von Natriumstearylfumarat zu dieser Mischung und Abmischen derselben, bis eine homogene Pulvermischung erhalten wird,
f. Komprimieren der abgemischten Mischung, um Tabletten zu bilden oder die Pulvermischung in Kapseln abzufüllen.

28. Direktkomprimierungsverfahren zur Zubereitung der pharmazeutischen Zusammensetzung nach einem der vorhergehenden Ansprüche umfassend die folgenden Schritte:
a. Mischen der Zusammensetzung I und von Hydrochlorothiazid mit mikrokristalliner Cellulose, Natriumstärkeglycolat, Crospovidon und kolloidalem Siliciumdioxid während 15 bis 30 min.,
b. Hinzugeben von Natriumstearylfumarat zu dieser Mischung und Abmischen derselben, bis eine homogene Pulvermischung erhalten wird,
c. Komprimieren der endgültigen Pulvermischung, um Tabletten zu bilden oder die Pulvermischung in Kapseln abzufüllen.

29. Pharmazeutische Zusammensetzung nach einem der vorhergehenden Ansprüche zur Verhinderung oder Behandlung von Hypertonie in Säugetieren, insbesondere bei Menschen.

## Revendications

1. Composition pharmaceutique comprenant de l'acide 2-éthoxy-1-((2'-(5-oxo-2,5-dihydro-1,2,4-oxodiazol-3yl)-biphényl-4-yl)méthyl)-1H-benzimidazole-7-carboxylique (composé I) ou des sels pharmaceutiquement acceptables de celui-ci et un ou plusieurs diurétiques en tant que composants actifs, dans laquelle lesdits un ou plusieurs diurétiques sont sélectionnés parmi les dérivés thiazidiques, et la composition comprend en outre un désintégrant sélectionné dans le groupe comprenant la crospovidone, le glycolate d'amidon sodique, la croscarmellose sodique, l'hydroxypropylcellulose faiblement substituée ou les mélanges de ceux-ci.

2. Composition pharmaceutique selon la revendication 1, dans laquelle les dérivés thiazidiques sont sélectionnés dans le groupe comprenant l'hydrochlorothiazide, le méthylclothiazide, le benzylhydrochlorothiazide, le trichlorométhiazide, le cyclopenthiazide, le polythiazide, l'éthiazide, le cyclothiazide, le bendrofluméthiazide ou l'hydrofluméthiazide ou les mélanges de ceux-ci, de préférence le thiazide est l'hydrochlorothiazide.

3. Composition pharmaceutique selon les revendications 1 et 2, dans laquelle le composé I ou les sels pharmaceutiquement acceptables de celui-ci sont présents en une quantité entre 0,1 et 60,0 % en poids et l'hydrochlorothiazide est présent en une quantité de 0,1 à 40,0 % en poids.

4. Composition pharmaceutique selon les revendications 1 à 3, dans laquelle le sel pharmaceutiquement acceptable du composé I est le médoxomil.

5. Composition pharmaceutique selon la revendication 1, dans laquelle le désintégrant est la crospovidone et/ou le glycolate d'amidon sodique.

6. Composition pharmaceutique selon la revendication 5, dans laquelle la crospovidone est présente en une quantité entre 0,10 et 30,0 % en poids de la composition totale.

7. Composition pharmaceutique selon les revendications 1 à 6, dans laquelle le rapport pondéral entre la crospovidone et le glycolate d'amidon sodique se situe entre 30/1 et 1/100 en poids de la composition totale.

8. Composition pharmaceutique selon la revendication 1, dans laquelle le composé qui de plus contient un ou plusieurs excipients sont sélectionnés dans le groupe comprenant des charges et des diluants, des lubrifiants, des agents de glissement, des liants, des colorants, des agents d'enrobage ou des mélanges de ceux-ci.

9. Composition pharmaceutique selon la revendication 8, dans laquelle la charge et le diluant sont sélectionnés dans le groupe comprenant la cellulose microcristalline, le lactose, les sucres, les sels inorganiques, le phosphate de calcium dibasique dihydraté et similaire ou les mélanges de ceux-ci ; de préférence la charge et les diluants sont la cellulose microcristalline.

10. Composition pharmaceutique selon la revendication 8, dans laquelle le lubrifiant est sélectionné dans le groupe comprenant le fumarate de stéaryle sodique, le polyéthylène glycol, l'acide stéarique, les stéarates de métaux, le talc, l'acide borique, les huiles végétales hydrogénées, le benzoate et l'acétate de chlorure de sodium, le laurylsulfate de sodium ou de magnésium et similaire ou les mélanges de ceux-ci ; de préférence le lubrifiant est le fumarate de stéaryle sodique.

11. Composition pharmaceutique selon les revendications 9 et 10, dans laquelle le rapport pondéral entre la cellulose microcristalline et le fumarate de stéaryle sodique se situe entre 100/1 et 1/100 en poids de la formulation totale.

12. Composition pharmaceutique selon la revendication 8, dans laquelle l'agent de glissement est sélectionné dans le groupe comprenant le dioxyde de silicium colloïdal; des silicates tels que l'aluminium, le calcium et le magnésium ; le talc et similaire ou les mélanges de ceux-ci ; de préférence l'agent de glissement est le dioxyde de silicium colloïdal.

13. Composition pharmaceutique selon l'une quelconque des revendications précédentes, comprenant :
a. de 0,1 à 60,0 % de composé I ou de sels pharmaceutiquement acceptables de celui-ci
b. de 0,1 à 40,0 % d'hydrochlorothiazide
c. de 1,0 à 90,0 % de cellulose microcristalline
d. de 0,1 à 30,0 % de crospovidone
e. de 0,01 à 50,0 % de glycolate d'amidon sodique
f. de 0,01 à 20,0 % de fumarate de stéaryle sodique
g. de 0,01 à 15,0 % de dioxyde de silicium colloïdal.

14. Composition pharmaceutique selon la revendication 13, dans laquelle la composition pharmaceutique est exempte de stéarate de magnésium.

15. Composition pharmaceutique selon l'une quelconque des revendications précédentes, dans laquelle la forme pharmaceutique finale est administrée par voie orale sous la forme de comprimés, de comprimés double couche, de gélules, de poudres, ou de sachets.

16. Composition pharmaceutique selon la revendication 15, dans laquelle la forme pharmaceutique finale est un comprimé.

17. Composition pharmaceutique selon la revendication 16, dans laquelle le comprimé comprend facultativement une couche d'enrobage.

18. Composition pharmaceutique selon la revendication 16, dans laquelle le comprimé se présente sous la forme d'un comprimé double couche ayant le composé 1 ou des sels pharmaceutiquement acceptables de celui-ci dans une couche et de l'hydrochlorothiazide dans la seconde couche.

19. Composition pharmaceutique selon la revendication 18, dans laquelle le comprimé double couche comprend facultativement une couche d'enrobage.

20. Composition pharmaceutique selon la revendication 15, dans laquelle la forme pharmaceutique finale est une gélule.

21. Composition pharmaceutique selon les revendications 1 à 20, dans laquelle la forme pharmaceutique finale a une uniformité de contenu inférieure à un CV (coefficient de variation) de 2,0 %, de préférence inférieure à un CV de 1,0 %.

22. Composition pharmaceutique selon les revendications 1 à 19, dans laquelle la dureté du comprimé se situe entre 5 et 300 Newton, de préférence elle se situe entre 20 et 50 Newton.

23. Composition pharmaceutique selon les revendications 1 à 19, dans laquelle la friabilité du comprimé est inférieure à 1 %.

24. Composition pharmaceutique selon l'une quelconque des revendications précédentes, dans laquelle la composition pharmaceutique a une durée de conservation à long terme de 24 mois ou plus à température ambiante, dans son emballage d'origine.

25. Procédé de granulation par voie humide destiné à préparer la composition pharmaceutique selon l'une quelconque des revendications précédentes, comprenant les étapes suivantes :
a. la dissolution du composé I et de l'hydrochlorothiazide avec la moitié du glycolate d'amidon sodique et la moitié de la crospovidone dans un solvant organique pour former une solution,
b. tandis que la solution se mélange, la cellulose microcristalline est ajoutée et brassée dans un granulateur à cisaillement élevé pour former des granules,
c. le tamisage et le séchage des granules humides et le broyage des granules séchés,
d. l'addition de dioxyde de silicium colloïdal et du reste de glycolate d'amidon sodique et de crospovidone et leur mélange,
e. l'addition de fumarate de stéaryle sodique à ce mélange et leur brassage jusqu'à obtention d'un mélange poudreux homogène,
f. la compression du mélange brassé pour former des comprimés ou le remplissage des gélules avec le mélange poudreux.

26. Procédé de granulation par voie humide destiné à préparer la composition pharmaceutique selon l'une quelconque des revendications précédentes, comprenant les étapes suivantes :
a. la dissolution du composé I et de l'hydrochlorothiazide avec la cellulose microcristalline, la moitié du glycolate d'amidon sodique et la moitié de la crospovidone dans un solvant organique pour former une solution dans un granulateur à lit fluidisé,
b. le tamisage et le séchage des granules humides et le broyage des granules séchés,
c. l'addition de dioxyde de silicium colloïdal et du reste de glycolate d'amidon sodique et de crospovidone et leur mélange,
d. l'addition de fumarate de stéaryle sodique à ce mélange et leur brassage jusqu'à obtention d'un mélange poudreux homogène,
e. la compression du mélange brassé pour former des comprimés ou le remplissage des gélules avec le mélange poudreux.

27. Procédé de granulation par voie sèche destiné à préparer la composition pharmaceutique selon l'une quelconque des revendications précédentes, comprenant les étapes suivantes :
a. le mélange du composé I et de l'hydrochlorothiazide avec la cellulose microcristalline, la moitié du glycolate d'amidon sodique, la moitié de la crospovidone et la moitié du dioxyde de silicium colloïdal,
b. leur compression à l'aide d'un compacteur,
c. le tamisage de ce mélange poudreux,
d. l'addition du reste de cellulose microcristalline, de glycolate d'amidon sodique, de crospovidone et de dioxyde de silicium colloïdal et leur mélange,
e. l'addition de fumarate de stéaryle de sodium à ce mélange et leur brassage jusqu'à obtention d'un mélange poudreux homogène,
f. la compression du mélange brassé pour former des comprimés ou le remplissage des gélules avec le mélange poudreux.

28. Procédé de compression directe destiné à préparer la composition pharmaceutique selon l'une quelconque des revendications précédentes, comprenant les étapes suivantes :
a. le mélange du composé I et de l'hydrochlorothiazide avec la cellulose microcristalline, le glycolate d'amidon sodique, la crospovidone et le dioxyde de silicium colloïdal pendant 15 à 30 min,
b. l'addition de fumarate de stéaryle sodique à ce mélange et leur brassage jusqu'à obtention d'un mélange poudreux homogène,
c. la compression du mélange poudreux final pour former des comprimés ou le remplissage des gélules avec le mélange poudreux.

29. Composition pharmaceutique selon l'une quelconque des revendications précédentes, destinée à la prévention ou au traitement de l'hypertension chez les mammifères, en particulier chez les humains.
